# EUROPEAN PATENT SPECIFICATION

(11) **EP 1 363 869 B1**
(45) Date of publication and mention of the grant of the patent: **08.04.2015**
(21) Application number: 02701493.5
(22) Date of filing: 26.02.2002
(51) Int. Cl.: C07C 67/08, C07C 69/738

(54) **PROCESS FOR THE PREPARATION OF ALKYL BENZOYLACRYLATES**
VERFAHREN ZUR HERSTELLUNG VON ALKYLBENZOYLACRYLATEN
PROCEDE DE PREPARATION DE BENZOYLACRYLATES D'ALKYLE

(30) Priority: 26.02.2001 FR 0102572
(43) Date of publication of application: 26.11.2003
(73) Proprietor: WeylChem Lamotte, 60350 Trosly Breuil (FR)
(72) Inventor: SIDOT, Christian, F-60200 Compiegne (FR)
(74) Representative: Ackermann, Joachim
(86) International application number: PCT/IB2002/000638
(87) International publication number: WO 2002/067847

(56) References cited:
- EP-A- 0 494 620
- GIARDINA G A M ET AL: "REPLACEMENT OF THE QUINOLINE SYSTEM IN 2-PHENYL-4-QUINOLINECARBOXAMIDE NK-3 RECEPTOR ANTAGONISTS" FARMACO, SOCIETA CHIMICA ITALIANA, PAVIA, IT, vol. 54, no. 6, 1999, pages 364-374, XP000939369 ISSN: 0014-827X

## Description

The present invention relates to a process for the preparation of alkyl benzoylacrylates.

Alkyl benzoylacrylates and more particularly ethyl benzoylacrylate are useful products as intermediates in the pharmaceutical industry, and more particularly for the synthesis of Enalapril, an inhibitor of the angiotensin-converting enzyme.

Alkyl benzoylacrylates and more particularly ethyl benzoylacrylate can be prepared from benzoylacrylic acid, itself prepared from benzene and maleic anhydride (R. Delaby et al, Bull. Soc. Chim., 1961, p. 2061-64). They can also be prepared by reacting an alcoholate with 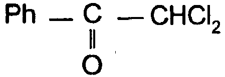 then reacting with triphenylcarbethoxymethylenephosphorane, and finally by sulphuric hydrolysis of the product obtained (C. Raulet and E. Levas, Bull. Soc. Chim. France, 1971, n°7, p. 2598-2606).

EP-A-0,494,620 describes the formation of aroylacrylic esters by reacting alkyl methyl ketones with acetal-esters or hemiacetal-esters of glyoxylic acid in a strong acid medium. These acetal-esters or hemiacetal-esters can be prepared in-situ in the reaction medium by reacting glyoxylic acid with an alcohol in a strong acid medium.

FR-A-2,504,127 describes the synthesis of trimethoxybenzoylacrylic acid or trimethoxybenzoyllactic acid by condensing glyoxylic acid or one of its alkyl esters with trimethoxyacetophenone.

In the article of G.A.M. Giardina et al. "Replacement of the quinoline system in 2-phenyl-4-quinolinecarboxamide NK-3 receptor antagonists" in IL FARMACO 54 (1999), 364-374, a synthesis of methyl 3-benzoylacrylate is disclosed by condensation of acetophenone with glyoxylic acid, followed by esterification and subsequent dehydration by p-toluenesulfonic acid in toluene at reflux.

More efficient processes for preparing alkyl benzoacrylates are always being sought.

For this reason, a subject of the present invention is a process for the preparation of alkyl benzoylacrylates of formula (I) 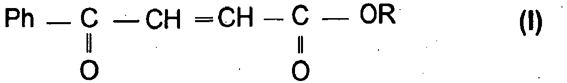 in which R is an alkyl group comprising 1 to 9 carbon atoms and Ph is a phenyl radical, characterized by the fact that the reaction is carried out in an acid medium, in the same reactor (so-called "one pot" reaction), without isolation of the intermediate products, by carrying out the following operations:
- condensation of acetophenone with glyoxylic acid in order to obtain a compound of formula (II) 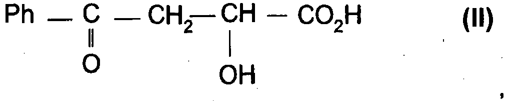
- elimination of a water/acetophenone mixture,
- elimination of the excess acetophenone,
- esterification of the compound of formula (II) with an alcohol of formula (III)

   R - OH (III)

   in which R is an alkyl group comprising 1 to 9 carbon atoms, in the presence of a strong acid, in order to obtain a compound of formula (IV) 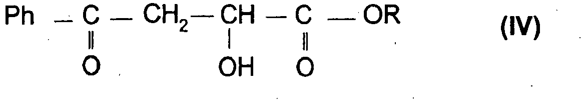 in which R and Ph have the same meaning as previously, without isolating the intermediate product,
- addition of a solvent,
- elimination of a mixture constituted by water, solvent and an alcohol of formula (III),
- dehydration of the compound of formula (IV) by azeotropic distillation of water,
- neutralization of the strong acid by a basic aqueous solution,
- decanting and concentration of the organic phase,
- recovery of the sought final product of formula (I).

R is a branched or preferably linear alkyl group. It preferably contains from 1 to 5 carbon atoms, and in particular from 1 to 3 carbon atoms. There can be mentioned for example the methyl, ethyl, n- and iso-propyl, n-, sec- and tert-butyl, n-pentyl radicals.

Under preferential conditions for the implementation of the above process, one or more of the following operations is carried out:
- 2 to 8 equivalents, and preferably 4 equivalents, of acetophenone are condensed with 1 equivalent of glyoxylic acid,
- the compound of formula (II) is esterified with 2 to 10 equivalents, preferably 3 equivalents, of an alcohol of formula (III) in the presence of a strong acid per equivalent of initial glyoxylic acid.

Under other preferential conditions of the invention
- the alcohol of formula III used is ethanol,
- the strong acid used is sulphuric acid,
- the azeotroping solvent is toluene,
- the ethyl benzoyllactate is dehydrated by azeotropic distillation after adding further toluene,
- the basic solution used is an aqueous solution of sodium bicarbonate.

Under other preferential implementation conditions, ethyl benzoylacrylate is prepared.

The process which is a subject of the present invention allows the direct preparation of alkyl benzoylacrylate starting from acetophenone and glyoxylic acid, without the intermediate isolation of benzoyllactic acid.

It is remarkably efficient in particular for the following reasons:
- the aldolization reaction between the acetophenone and the carbonyl function of the glyoxylic acid according to the invention produces benzoyllactic acid quantitatively 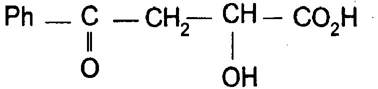 and the formation of the corresponding acrylic acid is minimized, 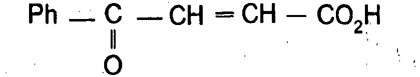
- the esterification of the benzoyllactic acid in the presence of an alcohol and a strong acid catalysis (sulphuric acid), initially, without elimination of the water formed, which allows the esterification of the benzoyllactic acid to alkyl benzoyllactate 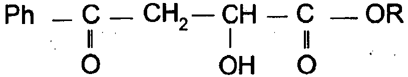 while avoiding dehydrating the alcohol function, which in turn avoids the formation of the compound of formula 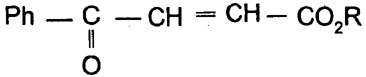
- the dehydration of the benzoyllactic acid to alkyl benzoylacrylate of formula 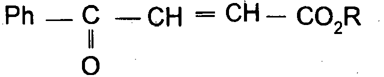 minimizes the addition of alcohol on the double bond and avoids the formation of the by-product

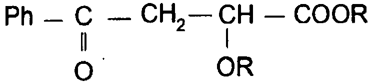

This set of conditions leads in particular to an excellent yield.

The present invention also has the advantage of being able to recycle, if desired, the acetophenone introduced at the beginning of the reaction from the acetophenone-water mixture distilled during the reaction, which decants at ambient temperature.

The following examples will allow a better understanding of the invention.

### EXAMPLE 1

The following are introduced into a reactor:
- 29.6 g of 50 % glyoxylic acid in water (0.2 mole),
- 96 g of acetophenone (0.8 mole). A heterogeneous reaction mixture is obtained the aqueous phase of which has an acid pH which is heated at 80°C under 50 millibars for 5 hours while eliminating 16.5 g of distillate comprising a water / acetophenone mixture 85/15.

The vacuum is then increased progressively to 2 millibars and the reaction medium is heated at 120°C for 8 hours in order to eliminate the excess acetophenone. The composition of the medium, determined by high performance liquid chromatography (HPLC), allows the determination of a yield of benzoyllactic acid of 92 % and of benzoylacrylic acid of 3 %. After returning to 70°C, the medium is diluted with 28 g of absolute ethanol (0.6 mole). The reaction medium is then heated under reflux for 2 hours in the presence of 0.34 g sulphuric acid. 75 g of toluene is then added and 40 g of a mixture of solvent constituted by water (10 %), toluene (45 %) and ethanol (45 %) is eliminated. Using HPLC analysis, it is noted that the reaction medium contains a great majority of ethyl benzoyllactate. Dehydration of the ethyl benzoyllactate to ethyl benzoylacrylate is carried out by azeotropic distillation of the water after adding 50 g of fresh toluene. 5.2 g of heavy phase is then eliminated constituted by 52 % water, 45 % ethanol and 3 % toluene. After neutralization of the sulphuric acid with an aqueous solution of sodium bicarbonate, decanting and concentration of the solvents, 44 g of a yellow oil is recovered containing 81 % ethyl benzoylacrylate (cis + trans), i.e. a yield of 87 % with respect to the starting glyoxylic acid.

### EXAMPLE 2

The following are introduced into a reactor:
- 593.2 g of 50 % glyoxylic acid in water (4 moles),
- 1,923 g of acetophenone (16 moles). A heterogeneous reaction mixture is obtained the aqueous phase of which has an acid pH, which is heated at 80°C under 50 millibars for 5 hours while eliminating 325.3 g of distillate comprising a water / acetophenone mixture 85/15.

The vacuum is then progressively increased to 2 millibars and the reaction medium is heated to 120°C for 8 hours in order to eliminate the excess acetophenone. The composition of the medium, determined by HPLC analysis, allows the determination of a yield of benzoyllactic acid of 89 % and of benzoylacrylic acid of 5 %. After returning to 70°C, the medium is diluted with 555.9 g of absolute ethanol (12 moles). The reaction medium is then heated under reflux for 2 hours in the presence of 7.1 g of sulphuric acid. 1501 g of toluene is then added and 796 g of a mixture of solvent constituted by water (9.1 %), toluene (45 %) and ethanol (46 %) is eliminated. Using HPLC analysis, it is noted that the reaction medium contains a great majority of ethyl benzoyllactate. Dehydration of the ethyl benzoyllactate to ethyl benzoylacrylate is carried out by azeotropic distillation of the water after adding 1213 g of fresh toluene. 110 g of heavy phase is then eliminated, constituted by approximately 50 % water and 50 % ethanol. After neutralization of the sulphuric acid using an aqueous solution of sodium bicarbonate, decanting and concentration of the solvents, 683 g of a yellow oil is recovered containing 98.9 % ethyl benzoylacrylate (cis + trans), i.e. a yield of 83 % with respect to the starting glyoxylic acid.

## Claims

1. Process for the preparation of alkyl benzoylacrylates of formula 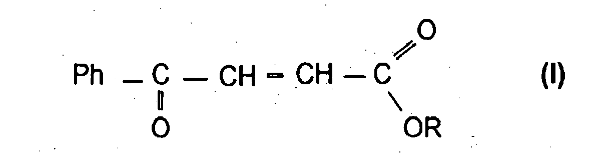 in which R is an alkyl group comprising 1 to 9 carbon atoms and Ph is a phenyl radical, **characterized in that** the reaction is carried out in an acid medium in the same reactor, without isolation of the intermediate products, by carrying out the following operations:
- condensation of 2 to 8 equivalents of acetophenone with 1 equivalent of glyoxylic acid in order to obtain a compound of formula (II) 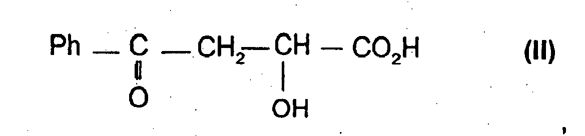
- elimination of a water/acetophenone mixture,
- elimination of the excess acetophenone,
- esterification of the compound of formula (II) with an alcohol of formula (III)
R-OH **(III)**
in which R is an alkyl group comprising 1 to 9 carbon atoms, in the presence of a strong acid, in order to obtain a compound of formula (IV) 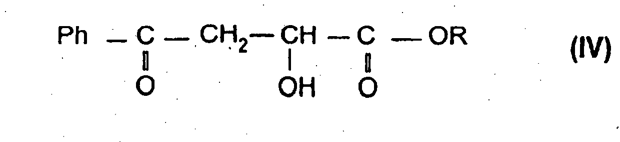 in which R and Ph have the same meaning as previously, without isolation of intermediate product,
- addition of a solvent,
- elimination of a mixture constituted by water, solvent and alcohol of formula (III),
- dehydration of the compound of formula (IV) by azeotropic distillation of the water,
- neutralization of the strong acid with a basic aqueous solution,
- decanting and concentration of the organic phase,
- recovery of the sought final product of formula (I).

2. Process according to claim 1, **characterized by** the fact that 1 equivalent of glyoxylic acid is reacted with 4 equivalents of acetophenone.

3. Process according to claim 1 or 2, **characterized by** the fact that 2 to 10 equivalents of alcohol are reacted per equivalent of initial glyoxylic acid.

4. Process according to one of claims 1 to 3, **characterized by** the fact that 3 equivalents of alcohol are reacted per equivalent of initial glyoxylic acid.

5. Process according to one of claims 1 to 4, **characterized by** the fact that the alcohol of formula III used is ethanol.

6. Process according to one of claims 1 to 5, **characterized by** the fact that the strong acid used is sulphuric acid.

7. Process according to one of claims 1 to 6, **characterized by** the fact that the azeotroping solvent used is toluene.

8. Process according to one of claims 1 to 7, **characterized by** the fact that the basic aqueous solution used is an aqueous solution of sodium bicarbonate.

9. Process according to one of claims 1 to 8, **characterized by** the fact that ethyl benzoylacrylate is prepared.

## Patentansprüche

1. Verfahren zur Herstellung von Benzoylacrylsäurealkylestern der Formel 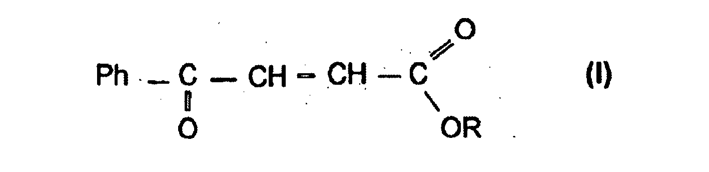 worin R für eine Alkylgruppe mit 1 bis 9 Kohlenstoffatomen steht und Ph für einen Phenylrest steht, **dadurch gekennzeichnet, dass** man die Umsetzung in einem sauren Medium in demselben Reaktor ohne Isolierung der Zwischenprodukte durchführt, indem man die folgenden Arbeitsgänge durchführt:
- Kondensation von 2 bis 8 Äquivalenten Acetophenon mit 1 Äquivalent Glyoxylsäure zum Erhalt einer Verbindung der Formel (II) 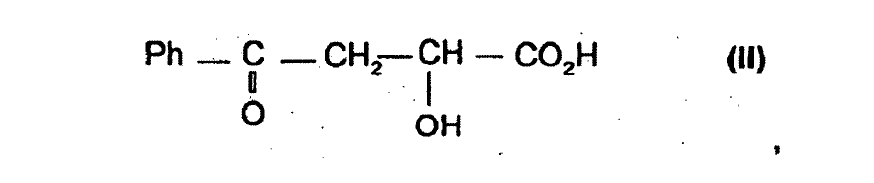
- Eliminierung einer Wasser/Acetophenon-Mischung,
- Eliminierung des überschüssigen Acetophenons,
- Veresterung der Verbindung der Formel (II) mit einem Alkohol der Formel (III)
**R-OH** **(III)**
- worin R für eine Alkylgruppe mit 1 bis 9 Kohlenstoffatomen steht, in Gegenwart einer starken Säure zum Erhalt einer Verbindung der Formel (IV) 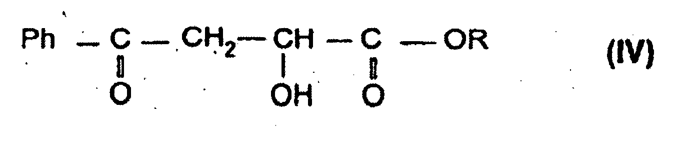 worin R und Ph die gleiche Bedeutung wie oben besitzen, ohne Isolierung des Zwischenprodukts,
- Zugabe eines Lösungsmittels,
- Eliminierung einer Mischung aus Wasser, Lösungsmittel und Alkohol der Formel (III),
- Dehydratisierung der Verbindung der Formel (IV) durch azeotrope Abdestillation des Wassers,
- Neutralisation der starken Säure mit einer basischen wässrigen Lösung,
- Dekantieren und Aufkonzentrieren der organischen Phase,
- Gewinnung des gesuchten Endprodukts der Formel (I).

2. Verfahren nach Anspruch 1, **dadurch gekennzeichnet, dass** 1 Äquivalent Glyoxylsäure mit 4 Äquivalenten Acetophenon umgesetzt werden.

3. Verfahren nach Anspruch 1 oder 2, **dadurch gekennzeichnet, dass** 2 bis 10 Äquivalente Alkohol pro Äquivalent eingesetzte Glyoxylsäure umgesetzt werden.

4. Verfahren nach einem der Ansprüche 1 bis 3, **dadurch gekennzeichnet, dass** 3 Äquivalente Alkohol pro Äquivalent eingesetzte Glyoxylsäure umgesetzt werden.

5. Verfahren nach einem der Ansprüche 1 bis 4, **dadurch gekennzeichnet, dass** es sich bei dem verwendeten Alkohol der Formel III um Ethanol handelt.

6. Verfahren nach einem der Ansprüche 1 bis 5, **dadurch gekennzeichnet, dass** es sich bei der verwendeten starken Säure um Schwefelsäure handelt.

7. Verfahren nach einem der Ansprüche 1 bis 6, **dadurch gekennzeichnet, dass** es sich bei dem verwendeten Azeotropierungslösungsmittel um Toluol handelt.

8. Verfahren nach einem der Ansprüche 1 bis 7, **dadurch gekennzeichnet, dass** es sich bei der basischen wässrigen Lösung um eine wässrige Lösung von Natriumhydrogencarbonat handelt.

9. Verfahren nach einem der Ansprüche 1 bis 8, **dadurch gekennzeichnet, dass** Benzoylacrylsäureethylester hergestellt wird.

## Revendications

1. Procédé de préparation de benzoylacrylates d'alkyle de formule 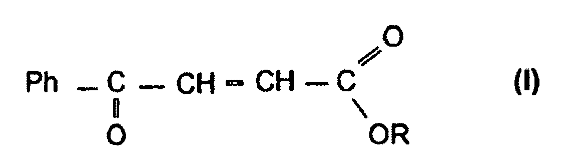 où R représente un groupement alkyle comprenant de 1 à 9 atomes de carbone et Ph représente un radical phényle, **caractérisé en ce que** la réaction est effectuée dans un milieu acide dans le même réacteur, sans isolement des produits intermédiaires, en effectuant les opérations suivantes :
- une condensation de 2 à 8 équivalents d'acétophénone avec 1 équivalent d'acide glyoxylique afin d'obtenir un composé de formule (II) 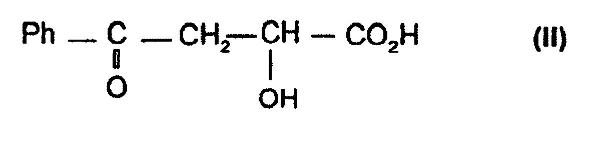
- une élimination d'un mélange d'eau/acétophénone,
- une élimination d'un excès d'acétophénone,
- une estérification du composé de formule (II) avec un alcool de formule (III)
R-OH **(III)**
où R représente un groupement alkyle comprenant de 1 à 9 atomes de carbone, en présence d'un acide fort, afin d'obtenir un composé de formule (IV) 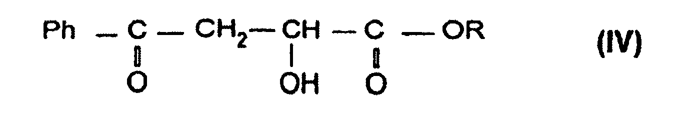 où R et Ph représentent les mêmes groupements que précédemment, sans isolement de produit intermédiaire,
- un ajout d'un solvant,
- une élimination d'un mélange constitué d'eau, de solvant et d'un alcool de formule (III),
- une déshydratation du composé de formule (IV) par distillation azéotropique de l'eau,
- une neutralisation de l'acide fort avec une solution aqueuse basique,
- une décantation et une concentration de la phase organique,
- une récupération du produit final recherché de formule (I).

2. Procédé selon la revendication 1, **caractérisé par le fait que** 1 équivalent d'acide glyoxylique est introduit pour réaction avec 4 équivalents d'acétophénone.

3. Procédé selon la revendication 1 ou 2, **caractérisé par le fait que** de 2 à 10 équivalents d'alcool sont introduits pour réaction par équivalent d'acide glyoxylique initial.

4. Procédé selon l'une des revendications 1 à 3, **caractérisé par le fait que** 3 équivalents d'alcool sont introduits pour réaction par équivalent d'acide glyoxylique initial.

5. Procédé selon l'une des revendications 1 à 4, **caractérisé par le fait que** l'alcool de formule III utilisé est l'éthanol.

6. Procédé selon l'une des revendications 1 à 5, **caractérisé par le fait que** l'acide fort utilisé est l'acide sulfurique.

7. Procédé selon l'une des revendications 1 à 6, **caractérisé par le fait que** le solvant azéotropant utilisé est le toluène.

8. Procédé selon l'une des revendications 1 à 7, **caractérisé par le fait que** la solution aqueuse basique utilisée est une solution aqueuse de bicarbonate de sodium.

9. Procédé selon l'une des revendications 1 à 8, **caractérisé par le fait que** le benzoylacrylate d'éthyle est préparé.
